# EUROPEAN PATENT SPECIFICATION

(11) **EP 2 883 560 B1**
(45) Date of publication and mention of the grant of the patent: **31.05.2017**
(21) Application number: 12882685.6
(22) Date of filing: 10.08.2012
(51) Int. Cl.: A61M 5/145

(54) **SYRINGE PUMP**
SPRITZENPUMPE
POMPE À SERINGUE

(43) Date of publication of application: 17.06.2015
(73) Proprietor: Terumo Kabushiki Kaisha, Tokyo 151-0072 (JP)
(72) Inventor: NAKANISHI, Masaru, Fujinomiya-shi Shizuoka 418-0015 (JP)
(74) Representative: Casalonga
(86) International application number: PCT/JP2012/005123
(87) International publication number: WO 2014/024235

(56) References cited:
- EP-A1- 0 198 734
- JP-A- H0 542 214
- JP-A- 2001 259 034
- JP-A- 2009 291 323
- US-A1- 2007 210 535

## Description

### Technical Field

The present invention relates to a syringe pump to which a syringe is attached and which is for sending a medicinal solution in the syringe to a patient. More specifically, the present invention relates to an infusion device according to the preamble of claim 1, such as it is, e.g., known from JPH05-42214.

### Background Art

A syringe pump is used, for example, in an intensive-care unit (ICU) and is used for performing, on a patient, treatment of sending a medicinal solution such as an anticancer drug, an anesthetic, a chemotherapeutic agent, blood, or a nutritional supplement accurately for a relatively long period of time. Compared to other infusion pumps, control of a flow rate of a medicinal solution (control of injection volume (mL) or injection rate (mL/h)) by a syringe pump is precise and superior.

In a treatment room or an operating room in which a syringe pump is used, a plurality of kinds of syringes having different capacity is prepared in advance. A healthcare professional selects a syringe having necessary capacity from the plurality of kinds of syringes and attaches the syringe of the selected capacity to the above-described syringe pump. When the syringe is attached to a housing part of the syringe pump, a body flange can be gripped. When a motor of the syringe pump is driven, a slider included as a moving member pushes a pusher flange of a syringe pusher toward a side of a syringe body by degrees, whereby a medicinal solution in the syringe body can be sent to a patient through a tube (see Patent Literature 1).

### Citation List

### Patent Literature

Patent Literature 1: JP 2010-88564 A

### Summary of Invention

### Technical Problem

Incidentally, a slider of a syringe pusher driving part includes a structure in which a syringe pusher is pushed to a side of a syringe body by a feed screw rotated by a motor. The feed screw is covered by a bellows-shaped cover member. The cover member is arranged between the slider of the syringe pusher driving part and a housing part. When the syringe pusher is pushed toward the side of the syringe body, the slider moves in a direction closer to the syringe body. Thus, the cover member is pushed by the slider and is elastically deformed and contracted. The cover member is formed by successively forming a plurality of ridge parts. The ridge parts include a uniform outline dimension.

However, in a case where the ridge parts of the cover member include a uniform outline dimension, each ridge part is large. Thus, when the cover member is extended, a long length of extension can be secured. However, in a state in which the cover member is the most contracted, a length of contraction of when the cover member the most contracted becomes long. Thus, it is not possible to reduce the length of contraction. Since the cover member is placed between the slider and the housing part, a distance to a position where the slider becomes the closest to a side of the housing part becomes long. Thus, in order to secure a necessary movement amount of the slider, that is, a pushing amount necessary for the syringe pusher, a chassis of the syringe pump becomes large.

Thus, a purpose of the present invention is to provide a syringe pump in which a chassis can be downsized by reducing a length of a contracted cover member and by reducing a distance between a moving member of a syringe pusher driving part and a housing part in a case where the moving member is the closest to a side of the housing part.

### Solution to Problem

A syringe pump of the present invention is defined in claim 1. It is a syringe pump to which a syringe filled with a medicinal agent is attached and which is configured to send the medicinal agent in the syringe to a patient, including: a syringe setting part configured to set a syringe body of the syringe; and a syringe pusher driving part configured to push a syringe pusher of the syringe, wherein the syringe pusher driving part includes a moving member configured to grip the syringe pusher detachably and to push out the medicinal agent in the syringe body by moving the syringe pusher relative to the syringe body, a guiding member configured to move and guide the moving member to a side of the syringe body, and a cover member which covers the guiding member and which can be elastically deformed and contracted as the moving member moves to the side of the syringe body, the cover member includes protruded parts which are successive in a bellows-shape, the protruded parts include two first protruded parts which are provided successively in an identical outer diameter dimension and at least one second protruded part which is provided between the first protruded parts and which has an outer diameter dimension smaller than the outer diameter dimension of each of the first protruded parts, and an outer diameter dimension of a connection part between the two successively-formed first protruded parts is set smaller than an outer diameter dimension of a connection part between the second protruded part and the first protruded part.

It is preferable that one end part of the cover member is connected to a side surface part of the syringe setting part and the other end part of the cover member is connected to a side surface of the moving member.

According to the above configuration, it is possible to cover a whole guiding member in such a manner that the side surface part of the syringe setting part and the moving member are connected to each other.

It is preferable that a storage part configured to store at least a part of the contracted cover member is provided on the side surface of the moving member. According to the above configuration, since at least a part of the contracted cover member can be stored in the storage part of the moving member, a distance to a position where the moving member becomes the closest to the side of the housing part can be reduced.

It is preferable that the cover member includes a drip-proof structure and the guiding member is a feed screw to feed the moving member by being rotated by an operation of a motor. According to the above configuration, the cover member prevents a solution from the outside from attaching to a feed screw.

It is preferable that a display unit configured to display information and an operation panel unit including an operation button are arranged at an upper part of a body of the syringe pump and the syringe setting part and the syringe pusher driving part are arranged at a lower part of the body of the syringe pump.

According to the above configuration, it is possible for a healthcare professional to perform an operation of sending a medicinal solution from the syringe while checking information on the display unit at the upper part of the body. Then, it is possible for a healthcare professional to operate an operation button on the operation panel unit while checking information on the display unit at the upper part of the body.

### Advantageous Effects of Invention

In the present invention, it is possible to provide a syringe pump in which a chassis can be downsized by reducing a length in an axial direction of a contracted cover member and by reducing a distance between a moving member of a syringe pusher driving part and a housing part in a case where the moving member is the closest to a side of the housing part and in which the cover member can be stretched further more when the cover member is extended in the axial direction. In the syringe pump, a syringe of 2.5 mL to 50 mL can be used.

### Brief Description of Drawings

Fig. 1 is a perspective view illustrating a syringe pump of a first preferred embodiment of the present invention.
Fig. 2 is a perspective view in which the syringe pump illustrated in Fig. 1 is seen in a W-direction.
Fig. 3 (A) to Fig. 3 (C) are perspective views illustrating an example of a plurality of sizes of syringes.
Fig. 4 is a view illustrating an electric configuration example in the syringe pump.
Fig. 5 is a perspective view illustrating a part of a syringe setting part and a syringe pusher driving part illustrated in Fig. 2.
Fig. 6 is a perspective view in which a part of the syringe setting part and the syringe pusher driving part illustrated in Fig. 5 is enlarged and seen in an E-direction.
Fig. 7 is an exploded perspective view illustrating the syringe pusher driving part, a boot, and the like.
Fig. 8 is a front view including a partial sectional surface illustrating an example of a shape of the boot.
Fig. 9 (A) and Fig. 9 (B) are perspective views illustrating a state to fix a pusher flange to a slider from a state in which the boot is the most extended.
Fig. 10(A) and Fig. 10(B) are perspective views illustrating a state in which the boot is contracted.
Fig. 11 (A) to Fig. 11 (C) are views illustrating a state in which the boot is the most contracted from the state in which the boot is the most extended.
Fig. 12 is a view illustrating a boot of a second embodiment of the present invention.

### Description of Embodiments

In the following, preferred embodiments of the present invention will be described in detail with reference to the drawings.

Note that embodiments described in the following are preferred detail examples of the present invention. Thus, various technically-preferred limitations will be assigned. However, the scope of the present invention is not limited to these embodiments unless there is description to limit the present invention in the following description.

### First Embodiment

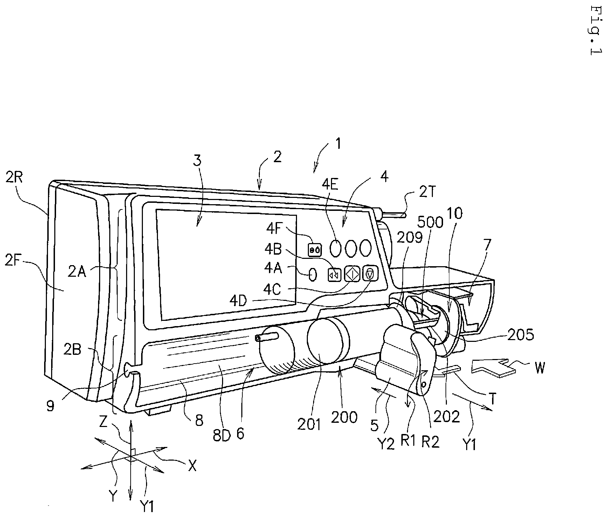

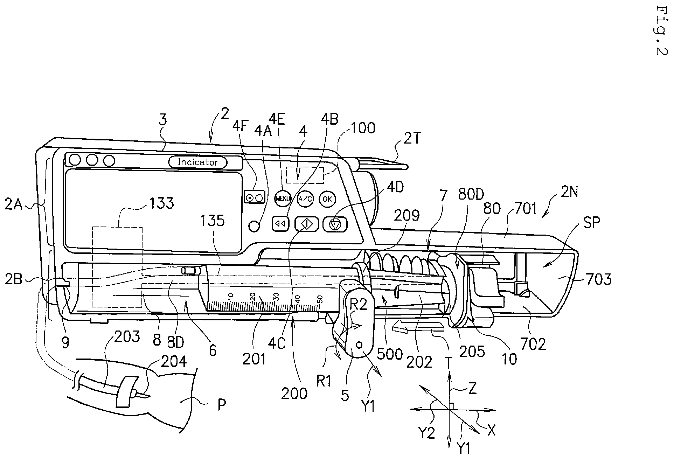

Fig. 1 is a perspective view illustrating a syringe pump of a first embodiment of the present invention. Fig. 2 is a perspective view in which the syringe pump illustrated in Fig. 1 is seen in a W-direction. The syringe pump 1 illustrated in Fig. 1 and Fig. 2 is a minute-amount continuous infusion pump which is used, for example, in an intensive-care unit and which is used for performing, on a patient, minute-amount infusion treatment of a medicinal solution such as an anticancer drug, an anesthetic, a chemotherapeutic agent, blood, or a nutritional supplement.

As illustrated in Fig. 1 and Fig. 2, for example, a syringe body 201 of a syringe 200 filled with a medicinal solution can be attached in an immovable manner and can be fixed to the syringe pump 1 by using a clamp 5. When a feed screw 135 is rotated by a motor 133 of a syringe pusher driving part 7 illustrated in Fig. 2, a syringe pusher pushing member 10 of the syringe pusher driving part 7 can push a syringe pusher 202 of the syringe 200 in a T-direction toward a side of the syringe body 201. Accordingly, as illustrated in Fig. 2, the medicinal solution in the syringe body 201 is sent to a patient P accurately through a tube 203 and an indwelling needle 204.

The syringe pusher pushing member 10 is an example of a moving member to push and move a syringe pusher 205 to the side of the syringe body 201 and is also referred to as a slider.

As illustrated in Fig. 2, the syringe pump 1 includes a chassis 2. The chassis 2 is integrally molded from a molded resin material having chemical resistance. As illustrated in Fig. 1, a front cover 2F and a rear cover 2R are joined and assembled, whereby the chassis 2 is configured as a chassis having liquid-tightness. Accordingly, as described later, a drip-proof (water-proof) structure with which it is possible to prevent a medicinal solution, liquid, or the like from entering an inside of the syringe pump 1 even when a medicinal solution, liquid, or the like spatters is included.

First, each element arranged in the chassis 2 of the syringe pump 1 will be described.

As illustrated in Fig. 2, the syringe pump 1 includes a chassis 2 and a handle 2T which is telescopic in a horizontal direction (X-direction). In an upper part 2A of the chassis 2, a display unit 3 and an operation panel unit 4 are arranged. In a lower part 2B of the chassis 2, a syringe setting part 6 and the syringe pusher driving part 7 are arranged. Accordingly, it is possible for a healthcare professional to perform an operation of sending a medicinal solution from the syringe 200 while visually checking information contents displayed in color on the display unit 3 in the upper part 2A of the chassis 2. Then, it is possible for the healthcare professional to operate an operation button of the operation panel unit 4 while checking information contents displayed in color on the display unit 3 of the chassis 2.

The display unit 3 illustrated in Fig. 1 and Fig. 2 is a color liquid crystal display apparatus (LCD) which is capable of performing a color graphic display. The display unit 3 is arranged in an upper left of the upper part 2A of the chassis 2 and on an upper side of the syringe setting part 6 and the syringe pusher driving part 7. In the upper part 2A of the chassis 2, the operation panel unit 4 is arranged on a right side of the display unit 3. In the illustrated example, on the operation panel unit 4, a pilot lamp 4A, a fast-forward switch button 4B, a start switch button 4C, a stop switch button 4D, a menu selection button 4E, a power switch 4F, and the like are arranged as operation buttons.

The upper part 2A of the chassis 2 illustrated in Fig. 2 is an upper half of the chassis 2. The lower part 2B of the chassis 2 is a lower half of the chassis 2. In the example illustrated in Fig. 1 and Fig. 2, the syringe setting part 6 and the syringe pusher driving part 7 are arranged in the X-direction. For example, the syringe setting part 6 can select a syringe 200 of necessary capacity from a plurality of kinds of syringes having different capacity and can embed and attach the selected syringe 200 detachably.

The syringe setting part 6 illustrated in Fig. 1 and Fig. 2 includes a housing part 8 to house the syringe body 201, the clamp 5, and a body flange pressing part 500 to embed and grip a body flange 209 detachably. The housing part 8 includes a recessed syringe body holding part 8D. On a wall part at an end part on a left side of the housing part 8, a tube fixing part 9 to sandwich the tube 203 detachably is formed. As illustrated in Fig. 2, the tube fixing part 9 is a groove part to sandwich and fix a part of the tube 203.

In Fig. 1 and Fig. 2, when a clamp 5 is operated by a healthcare professional and the syringe 200 is detached from the syringe setting part 6, for example, the clamp 5 is pulled in a Y1-direction (front direction) against force of a spring (not illustrated) and is rotated for 90 degrees in an R1-direction, whereby the clamp 5 is detached from an outer peripheral surface of the syringe body 201. Accordingly, the syringe body 201 can be released from the fixation by the clamp 5 and removed from the syringe body holding part 8D of the housing part 8. Also, the tube 203 can be removed from the tube fixing part 9.

Also, in a case where the clamp 5 is operated and the syringe 200 is housed into and attached to the housing part 8 of the syringe setting part 6, the clamp 5 is pulled in the Y1-direction against the force of the spring (not illustrated), is rotated for 90 degrees in an R2-direction, and is returned in a Y2-direction by the fore of the spring. Thus, the syringe body 201 can be housed into the syringe body holding part 8D of the housing part 8 and can be fixed by the clamp 5 while the tube 203 is embedded in the tube fixing part 9.

As illustrated in Fig. 1 and Fig. 2, when the syringe body 201 is housed into and attached to the syringe body holding part 8D of the housing part 8, the syringe pusher 202 is arranged in the syringe pusher driving part 7. The syringe pusher driving part 7 includes the syringe pusher pushing member 10. When the motor 133 in Fig. 2 is driven according to an instruction from the control unit and the feed screw 135 is rotated, the syringe pusher pushing member 10 pushes the pusher flange 205 of the syringe pusher 202 in the T-direction toward the side of the syringe body 201. Accordingly, the medicinal solution in the syringe body 201 can be sent to the patient P accurately through the tube 203 and the indwelling needle 204 for a relatively long period of time. Note that the X-direction, the Y-direction, and a Z-direction in Fig. 1 and Fig. 2 are orthogonal to each other. The Z-direction is an up-down direction.

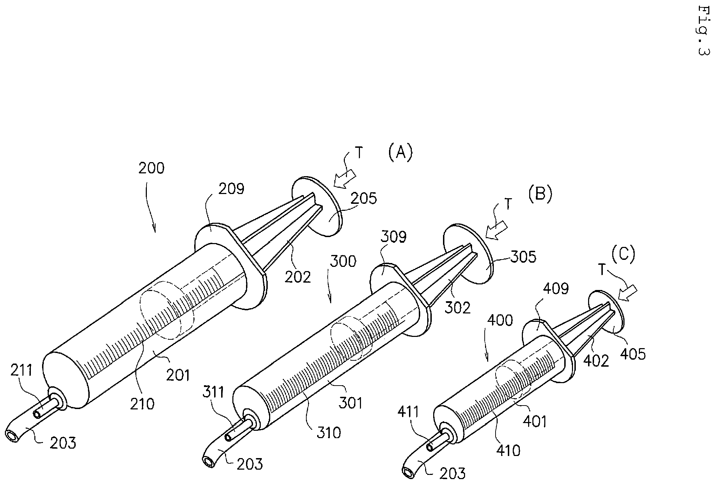

Fig. 3 (A) to Fig. 3 (C) are perspective views illustrating an example of a plurality of sizes of syringes (such as 2.5 mL syringe, 5 mL syringe, 10 mL syringe, 20 mL syringe, 30 mL syringe, and 50 mL syringe). In Fig. 1 and Fig. 2, for example, a syringe having the largest capacity of a medicinal solution, such as a 50 mL syringe 200 is fixed. The syringe 200 having the largest capacity of a medicinal solution which syringe is illustrated in Fig. 3 (A) includes the syringe body 201 and the syringe pusher 202. The syringe body 201 includes the body flange 209. The syringe pusher 202 includes the pusher flange 205. On the syringe body 201, a scale 210 of a medicinal solution is formed. At an outlet part 211 of the syringe body 201, one end part of the flexible tube 203 is connected detachably.

A syringe having a middle capacity of a medicinal solution which syringe is illustrated in Fig. 3 (B), such as a 10 mL syringe, a 20 mL syringe, or a 30 mL syringe 300 includes a syringe body 301 and a syringe pusher 302. The syringe body 301 includes a body flange 309. The syringe pusher 302 includes a pusher flange 305. On the syringe body 301, a scale 310 of a medicinal solution is formed. To an outlet part 311 of the syringe body 301, one end part of the flexible tube 203 is connected detachably.

A syringe having the smallest capacity of a medicinal solution which syringe is illustrated in Fig. 3(C), such as a 2.5 mL syringe or a 5 mL syringe 400 includes a syringe body 401 and a syringe pusher 402. The syringe body 401 includes a body flange 409 and the syringe pusher 402 includes a pusher flange 405. On the syringe body 401, a scale 410 of a medicinal solution is formed. To an outlet part 411 of the syringe body 401, one end part of the flexible tube 203 is connected detachably.

Capacity of a medicinal solution in the syringe 200 illustrated in Fig. 3(A) is, for example, 50 mL. Capacity of a medicinal solution in the syringe 300 illustrated in Fig. 3 (B) is, for example, 10 mL, 20 mL, or 30 mL. Capacity of a medicinal solution in the syringe 400 illustrated in Fig. 3(C) is, for example, 2.5 mL or 5 mL.

As illustrated in Fig. 3(A) to Fig. 3(C), sizes of the syringe bodies 201, 301, and 401 of the syringes 200, 300, and 400 are different from each other.

Similarly to the syringe 200 illustrated in Fig. 1 and Fig. 2, each of the syringe bodies 301 and 401 of the syringes 300 and 400 can be housed into and attached to the syringe body holding part 8D of the housing part 8. Three kinds of syringes are illustrated in Fig. 3(A) to Fig. 3(C) but are not the limitation. Capacity of a medicinal solution in a syringe is 2.5 mL to 50 mL and may be, for example, 20 mL, 30 mL, or 50 mL. Capacity in a syringe which can be set with respect to the syringe pump 1 can be selected arbitrarily.

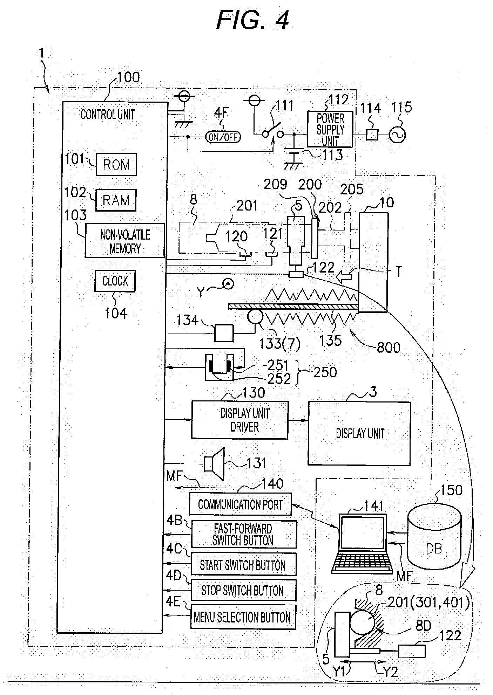

Next, with reference to Fig. 4, an electric configuration example in the syringe pump 1 illustrated in Fig. 1 and Fig. 2 will be described. In Fig. 4, the syringe pump 1 includes a control unit (computer) 100 to control a general operation. The control unit 100 is, for example, a microcomputer in one chip and includes a read-only memory (ROM) 101, a random access memory (RAM) 102, a non-volatile memory 103, and a clock 104. The clock 104 can correct current time by a predetermined operation. It is possible, for example, to acquire current time, to measure elapsed time of a predetermined solution-sending operation, and to measure reference time for control of a speed of sending a solution.

To the control unit 100 illustrated in Fig. 4, the power switch button 4F and a switch 111 are connected. By switching the power converter unit 112 with a rechargeable battery 113 such as a lithium-ion battery, the switch 111 supplies power to the control unit 100 from either of the power converter unit 112 and the rechargeable battery 113. The power converter unit 112 is connected to a commercial AC power source 115 via a plug 114. In Fig. 4, for example, a pair of detection switches 120 and 121 is arranged in the housing part 8. Each of the detection switches 120 and 121 preferably detects whether the syringe body 201 of the syringe 200 is arranged appropriately in the housing part 8 and gives notification to the control unit 100.

A potentiometer 122 which is illustrated in Fig. 4 and which is included as a clamp sensor is coupled to the clamp 5. The potentiometer 122 detects a movement amount of the clamp 5 in a case where the clamp 5 clamps the syringe body 201 and moves in the Y2-direction. Accordingly, information indicating the syringe body 201 (301 or 401) of which capacity is clamped by the clamp 5 is notified to the control unit 100 by using a detection signal. The control unit 100 acquires the movement amount in the Y-direction of the clamp 5 by the detection signal from the potentiometer 122 and can determine, for example, which of syringes respectively including the plurality of kinds of syringe bodies 201, 301, and 401 illustrated in Fig. 3(A) to Fig. 3(C) is attached.

When being driven by a motor driver 134 according to an instruction from the control unit 100, the motor 133 of the syringe pusher driving part 7 illustrated in Fig. 4 rotates the feed screw 135 and moves the syringe pusher pushing member 10 in the T-direction. Accordingly, the syringe pusher pushing member 10 pushes the syringe pusher 202 in the T-direction and sends the medicinal solution in the syringe body 201 illustrated in Fig. 2 to the patient P through the tube 203 and the indwelling needle 204 accurately.

In Fig. 4, a display unit driver 130 drives the display unit 3 according to an instruction from the control unit 100 and displays various kinds of information, notification contents, or the like. A speaker 131 announces various notification contents with sound according to an instruction from the control unit 100. The control unit 100 can perform interactive communication with an external computer 141 such as a desktop computer through a communication port 140. The external computer 141 is connected to a medicinal solution database (DB) 150. Medicinal solution information MF stored in the medicinal solution database 150 can be acquired by the control unit 100 through the external computer 141 and can be stored into the non-volatile memory 103 of the control unit 100. Based on the stored medicinal solution information MF, the control unit 100 displays the medicinal solution information MF or the like on the display unit 3.

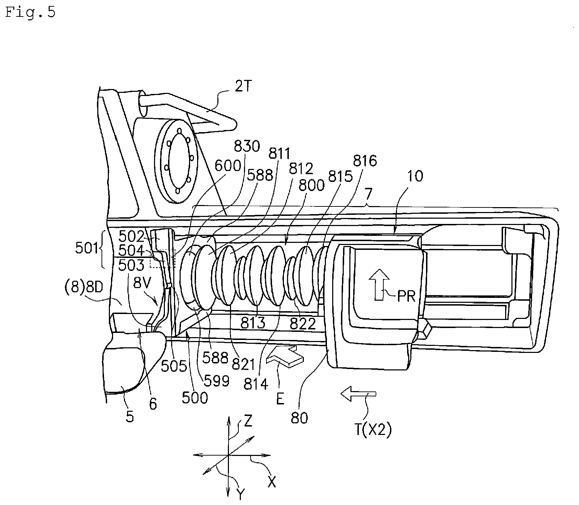

In Fig. 4, the fast-forward switch button 4B, the start switch button 4C, the stop switch button 4D, the menu selection button 4E, and the power switch 4F ate electrically connected to the control unit 100. In addition, to the control unit 100, a photocoupler sensor 250 which is included as a detector to detect that the body flange 209 is gripped by the body flange pressing part 500 (see Fig. 5) is electrically connected. The photocoupler sensor 250 includes a light emitting element 251 and a light receiving element 252 to receive light from the light emitting element 251.

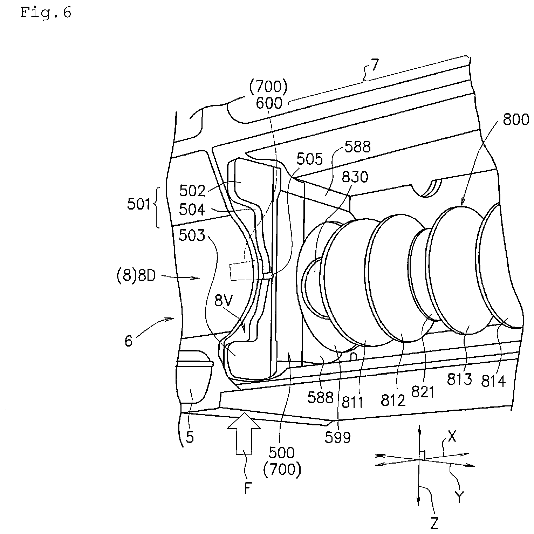

Next, with reference to Fig. 5 and Fig. 6, a detail structure of the syringe setting part 6 will be described. Fig. 5 is a perspective view illustrating a part of the syringe setting part 6 and the syringe pusher driving part 7 illustrated in Fig. 2. Fig. 6 is a perspective view in which the part of the syringe setting part 6 and the syringe pusher driving part 7 illustrated in Fig. 5 is enlarged and seen in an E-direction.

The syringe setting part 6 illustrated in Fig. 5 includes the housing part 8 to house the syringe body 201, the clamp 5, the body flange pressing part 500 to press and grip the body flange 209 (see Fig. 3 (A)) of the syringe 200, and the body flange detection unit 600.

As illustrated in Fig. 1 and Fig. 2, for example, the syringe body 201 of the syringe 200 is set in the syringe setting part 6 and the syringe body 201 of the syringe 200 is fixed by using the clamp 5. As illustrated in Fig. 5 and Fig. 6, the housing part 8 of the syringe setting part 6 is a recessed part which can house the syringe body 201. An axial direction of the housing part 8 is in the X-direction. A part of the outer peripheral surface of the syringe body 201 is close to an inner surface of the syringe body holding part 8D of the housing part 8 and the remaining part of the outer peripheral surface of the syringe body 201 is exposed to the outside. The body flange detection unit 600 includes the photocoupler sensor 250 illustrated in Fig. 4.

A shape of the body flange pressing part 500 will be described with reference to Fig. 5 and Fig. 6. The shape of the body flange pressing part 500 is just an example and a shape of the body flange pressing part 500 is not limited to the shape. The body flange pressing part 500 is arranged along a surface formed in the Y-direction and the Z-direction.

As illustrated in Fig. 5 and Fig. 6, the body flange pressing part 500 includes a leading end part 501 and two coupling parts 588. The leading end part 501 preferably includes two introducing parts 502 and 503 and a recessed part 504 formed between the introducing parts 502 and 503. Accordingly, as illustrated in Fig. 1 and Fig. 2, by using the two introducing parts 502 and 503, a healthcare professional can easily insert the body flange 209 of the syringe 200 in the Y2-direction between the inner surface of the body flange pressing part 500 and a right side surface part 8V of the syringe body holding part 8D. Thus, the body flange pressing part 500 can fix the body flange 209 securely. As illustrated in Fig. 5 and Fig. 6, between the coupling parts 588 on both sides, a substantially-circular hole part 599 is included. As illustrated in Fig. 6, the hole part 599 is formed in such a manner that a boot 800 which is included as a cover member and which will be described later passes therethrough. Accordingly, when the syringe pusher 202 illustrated in Fig. 2 is pushed toward the side of the syringe body 201 and the medicinal solution in the syringe body 201 is sent, the boot 800 can be elastically deformed and contracted without touching the body flange pressing part 500.

Referring back to Fig. 6, when the syringe pump 1 is used, the introducing part 502 is placed on an upper side in the Z-direction and the introducing part 503 is placed on a lower side. As illustrated in Fig. 6, it is preferable that a small recessed part 505 is further formed at a center position of the recessed part 504. The small recessed part 505 is a groove part into which a part of a blade part of the syringe pusher 202 illustrated in Fig. 2 is put while the syringe 200 is attached. Accordingly, the blade part of the syringe pusher 202 does not run on a surface of the recessed part 504 of the body flange pressing part 500. Thus, the syringe 200 can be securely fixed at a predetermined position. A case of the syringe 300 or 400 illustrated in Fig. 3(B) or Fig. 3(C) is also similar to the case of the syringe 200.

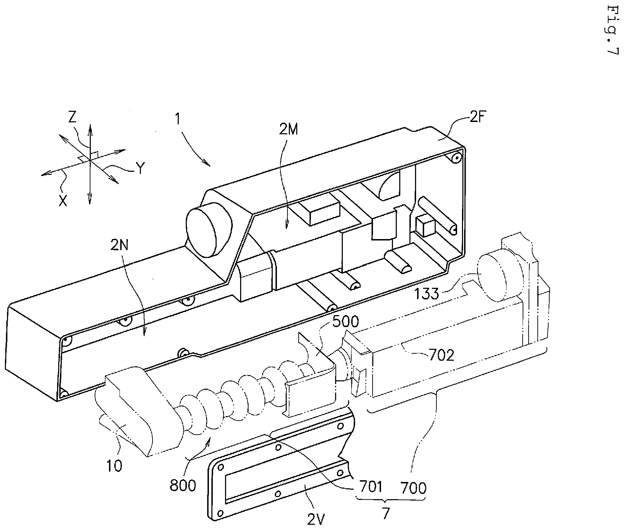

Fig. 7 is an exploded perspective view from a rear side illustrating the front cover 2F, the syringe pusher driving part 7, the body flange pressing part 500, the syringe pusher pushing member 10, and a cover member 2V. In Fig. 7, an inner surface side of the front cover 2F is illustrated. The front cover 2F includes a body housing part 2M and an extension part 2N extended from the body housing part 2M toward a side. In an inner part of the body housing part 2M and the extension part 2N, the syringe pusher driving part 7, the body flange pressing part 500, and the boot 800 are housed. To the extension part 2N, the cover member 2V is fixed by a screw.

The syringe pusher driving part 7 illustrated in Fig. 7 includes a driving part body 700, the syringe pusher pushing member 10, and the pushing operation unit 701 coupled to the syringe pusher pushing member 10. The driving part body 700 is housed in a lower part of the body housing part 2M of the front cover 2F. The pushing operation unit 701 and the syringe pusher pushing member 10 are housed in the extension part 2N.

Next, an example of a shape of the boot 800 which is illustrated in Fig. 5 and Fig. 6 and is included as a cover member will be described.

The boot 800 is a member which can be elastically deformed and contracted when the syringe pusher 202 illustrated in Fig. 2 is pushed to the side of the syringe body 201 and the medicinal solution in the syringe body 201 is sent. As illustrated in Fig. 5, the boot 800 is arranged between the right side surface part 8V of the syringe body holding part 8D of the housing part 8 and a cover member 80 of the syringe pusher pushing member 10. The boot 800 is made, for example, from silicone rubber, fluororubber, or thermoplastic elastomer which can be elastically deformed and contracted and which is not deteriorated by a medicinal solution or ultraviolet rays. The boot 800 can be extended and contracted as the syringe pusher pushing member 10 moves in the X1-direction and in an X2-direction.

The boot 800 is a drip-proof structure in order to cover a machine element such as the feed screw 135 illustrated in Fig. 4. Accordingly, even when the medicinal solution in the syringe body 201 spills, an infusion arranged on an upper side drops, or an antiseptic solution or the like used at the periphery spatters, attachment to the machine element such as the feed screw 135 is prevented.

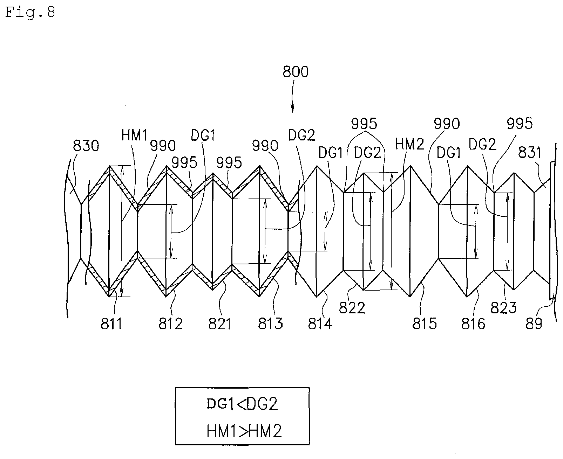

Fig. 8 is a front view including a partial sectional surface illustrating an example of a shape of the boot 800. As illustrated in Fig. 8 as an example, the boot 800 includes, for example, successive protruded parts which include a plurality of first protruded parts 811, 812, 813, 814, 815, and 816 having the same size (same outer diameter dimension) and a plurality of second protruded parts 821, 822, and 823 having an outer diameter smaller than that of the first protruded part and having the same size and which is bellows-shaped as a whole, and right and left coupling parts 830 and 831.

That is, the outer diameter of each of the second protruded parts 821, 822, and 823 is smaller than a diameter of the first protruded parts 811, 812, 813, 814, 815, and 816.

As illustrated in Fig. 6, the left coupling part 830 passes through the hole part 599 of the body flange gripping part 500 and is fixed to a side of the right side surface part 8V of the syringe body holding part 8D.

As illustrated in Fig. 8, on the left coupling part 830, two adjoining first protruded parts 811 and 812 are successively formed. Also, on the first protruded part 812, one second protruded part 821 is successively formed. On the one second protruded part 821, two adjoining first protruded parts 813 and 814 are successively formed. Also, on the first protruded part 814, one second protruded part 822 is successively formed. Moreover, on the one second protruded part 822, two adjoining first protruded parts 815 and 816 are successively formed. On the first protruded part 816, one second protruded part 823 is successively formed. The one second protruded part 823 is connected to a side of an inner side surface 89 of the cover member 80 through the right coupling part 831.

Accordingly, when the syringe pusher pushing member 10 moves to a left side and the boot 800 is elastically deformed and contracted, the second protruded parts 821, 822, and 823 enter the first protruded parts having a larger diameter. Note that in Fig. 8, the feed screw 135 and the like are not illustrated in order to simplify the drawing.

As illustrated in Fig. 8, an outer diameter dimension HM1 of an outermost part of each of the first protruded parts 811, 812, 813, 814, 815, and 816 is set larger than an outer diameter dimension HM2 of an outermost part of each of the second protruded parts 821, 822, and 823.

On the other hand, as illustrated in Fig. 8, an outer diameter dimension DG1 is set smaller than an outer diameter dimension DG2. More specifically, an outer diameter dimension DG1 of an innermost part in a connection part 990 between the first protruded parts 811 and 812, an outer diameter dimension DG1 of an innermost part in a connection part 990 between the first protruded parts 813 and 814, and an outer diameter dimension DG1 of an innermost part in a connection part 990 between the first protruded parts 815 and 816 are set smaller than an outer diameter dimension DG2 of an innermost part in a connection part 995 between the second protruded part 821 and the first protruded part 812, an outer diameter dimension DG2 of an innermost part in a connection part 995 between the second protruded part 821 and the first protruded part 813, an outer diameter dimension DG2 of an innermost part in a connection part 995 between the second protruded part 822 and the first protruded part 814, an outer diameter dimension DG2 of an innermost part in a connection part 995 between the second protruded part 822 and the first protruded part 815, and an outer diameter dimension DG2 of an innermost part in a connection part 995 between the second protruded part 823 and the first protruded part 816.

The outer diameter dimension DG1 is, for example, 12 mm to 14 mm and the outer diameter dimension DG2 is, for example, 14 mm to 16 mm. Also, DG1/DG2 is around 0.85.

Also, a thickness of each of the first protruded parts 811, 812, 813, 814, 815, and 816 and a thickness of each of the second protruded parts 821, 822, and 823 are around 0.5 mm.

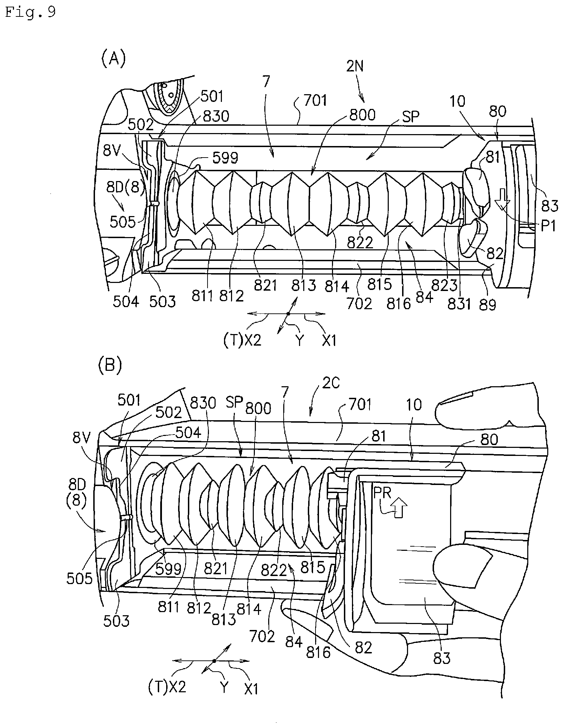

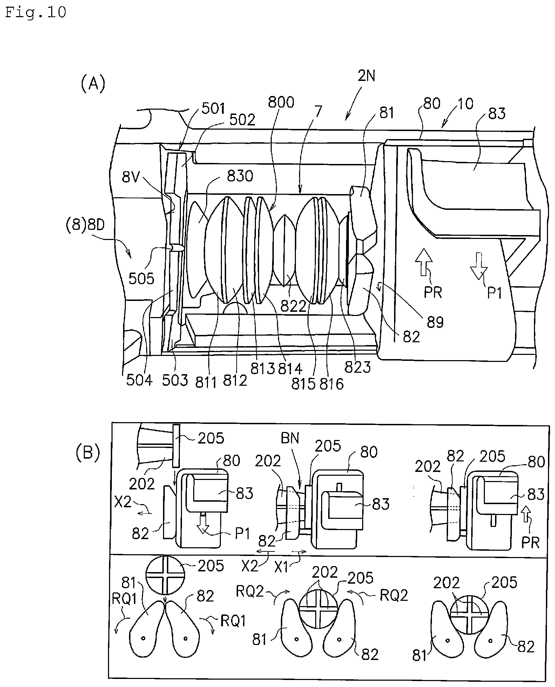

Next, a configuration example of the syringe pusher driving part 7 illustrated in Fig. 2 will be described with reference to Fig. 9 (A), Fig. 9 (B), Fig. 10 (A), and Fig. 10 (B). Fig. 9 (A) is a view illustrating a state in which the boot 800 is the most extended. Fig. 9 (B) is a view illustrating a state to fix the pusher flange 205 to the slider 10. Fig. 10(A) and Fig. 10(B) are perspective views illustrating a state in middle of contraction of the boot 800.

As illustrated in Fig. 2, Fig. 9(A), and Fig. 9(B), the syringe pusher driving part 7 is housed and held in the extension part 2N of a body cover 2. The extension part 2N is formed by being extended in the X1-direction from a lower part of the body cover 2. As illustrated in Fig. 2, the extension part 2N includes an upper side surface part 701, a lower side surface part 702, and a right side surface part 703. The extension part 2N includes a space SP surrounded by the upper side surface part 701, the lower side surface part 702, the right side surface part 703, and the right side surface part 8V of the syringe body holding part 8D of the housing part 8 which is illustrated in Fig. 9(A) and Fig. 9(B) and which has been already described. In the space SP, the syringe pusher driving part 7 is housed.

As illustrated in Fig. 2, according to an instruction from the control unit 100 in Fig. 4, the slider 10 of the syringe pusher driving part 7 pushes the pusher flange 205 of the syringe pusher 202 by degrees in the T-direction (X2-direction) relative to the syringe body 201. The feed screw 135 is an example of a guiding member to guide the slider 10 in the T (X2)-direction.

When being driven according to an instruction from the control unit 100, the motor 133 of the syringe pusher driving part 7 illustrated in Fig. 4 rotates the feed screw 135 and moves the slider 10 in the T-direction. Accordingly, the slider 10 can push the syringe pusher 202 in the T-direction and can send a medicinal agent in the syringe body 201 illustrated in Fig. 2 to the patient P through the tube 203 and the indwelling needle 204.

As illustrated in Fig. 9(A) and Fig. 9(B), the slider 10 includes the plastic cover member 80, two gripping members 81 and 82, and an operation lever 83. The cover member 80 can move in the X1-direction and in the X2-direction (T-direction) along a guide rail 84 of the extension part 2N. With a finger, a healthcare professional can push down the operation lever 83 in the P1-direction illustrated in Fig. 9(A) against biasing force or can lift the operation lever 83 in a PR-direction by the biasing force as illustrated in Fig. 9(B).

As illustrated in Fig. 10 (B), when the operation lever 83 is pushed down in the P1-direction by the healthcare professional, the gripping members 81 and 82 move in the X2-direction and away from the cover member 80 for a gap BN. When the operation lever 83 is pushed further more by the healthcare professional, the gripping members 81 and 82 are opened in RQ1-directions which are directions to be away from each other.

When the operation lever 83 is released by the healthcare professional after the pusher flange 205 of the syringe pusher 202 is embedded in the gap BN by the healthcare professional, the operation lever 83 is brought back in an RQ2-direction by force of the spring. Accordingly, the gripping members 81 and 82 move in the X1-direction by the force of the spring (not illustrated) and the pusher flange 205 is sandwiched and held between the gripping members 81 and 82 and the cover member 80. In addition, by closing the gripping members 81 and 82 in the RQ2-directions, the gripping members 81 and 82 can sandwich and hold the syringe pusher 202 from both sides.

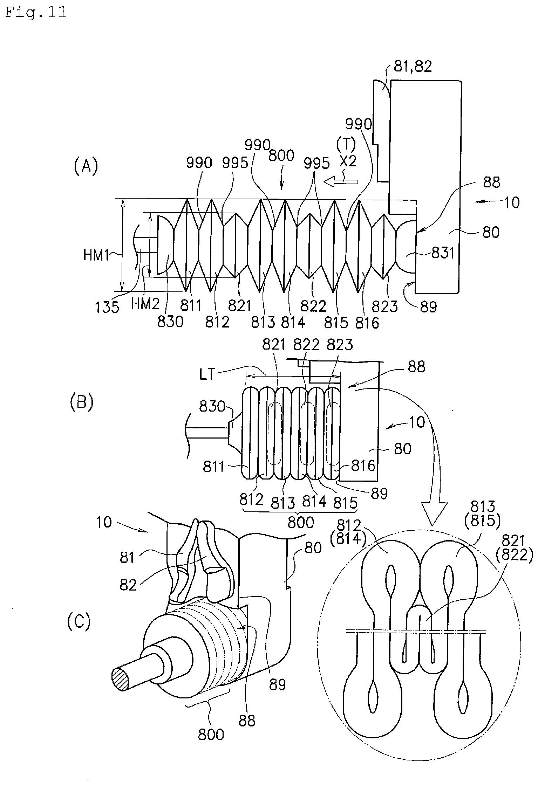

Fig. 11 (A) is a view illustrating a state in which the boot 800 is the most extended and Fig. 11 (B) is a state in which the boot 800 is the most contracted. Fig. 11(A) is a view illustrating a bottom surface side of the boot 800 and the slider 10. The outer diameter dimension HM2 of each of the second protruded parts 821, 822, and 823 is set smaller than the outer diameter dimension HM1 of each of the first protruded parts 811, 812, 813, 814, 815, and 816. The cover member 80 of the slider 10 includes a recessed boot storage part 88.

Fig. 11(B) and Fig. 11(C) are views illustrating a state in which the boot 800 is the most contracted. A part of the contracted boot 800 can be preferably stored in the boot storage part 88 of the cover member 80. Accordingly, at least a part of the contracted boot 800 which is the cover member can be housed in the boot storage part 88 of the slider 10 which is the moving member. Thus, it is possible to reduce a distance to a position where the slider 10 becomes the closest to the side of the housing part 8.

Here, a contraction function of the boot 800 will be described.

When the slider 10 pushes the syringe pusher in the X2-direction (T-direction) as illustrated in Fig. 11(A), the adjoining first protruded part 812 and first protruded part 813 can be stored with the one second protruded part 821 placed therebetween being folded as illustrated in Fig. 11(B). Similarly, the adjoining first protruded part 814 and first protruded part 815 can be stored with the one second protruded part 822 placed therebetween being folded. In addition, the remaining one second protruded part 823 can be stored, in a folded state, in the first protruded part 816. As illustrated in Fig. 11 (B), in a state in which the boot 800 is stored, the one second protruded part 821 can be kept folded (stored) between extended parts of the two folded first protruded parts 812 and 813. Similarly, the second protruded part 822 can be kept folded (stored) between the folded first protruded parts 814 and 815.

Next, an example of usage of the syringe pump 1 of the embodiment of the present invention will be described. A healthcare professional selects, for example, the syringe 200 among the plurality of kinds of syringes 200, 300, and 400 illustrated in Fig. 3 (A) to Fig. 3 (C) and attaches the syringe 200 to the syringe pump 1 as illustrated in Fig. 1 and Fig. 2. The syringe body 201 is housed in the syringe body holding part 8D of the housing part 8 by the healthcare professional and the tube 203 is fixed by the clamp 5 while being embedded in the tube fixing part 9. Accordingly, the syringe body 201 can be fixed to the syringe body holding part 8D of the housing part 8. In addition, a part of the body flange 209 is sandwiched and gripped between the right side surface part 8V and the body flange pressing part 500. Accordingly, as illustrated in Fig. 1 and Fig. 2, the syringe 200 can be gripped by using the body flange 209.

As illustrated in Fig. 9(A) and Fig. 9(B), while the operation lever 83 is pushed in the P1-direction with a finger, the syringe pusher pushing member 10 is pushed in the T-direction (X2-direction) by the healthcare professional and is brought to a state illustrated in Fig. 10 (B). As illustrated in Fig. 2, the syringe pusher pushing member 10 becomes closer to the body part 80 of the syringe pusher pushing member 10 and the two gripping members 81 and 82 of the slider 10 illustrated in Fig. 9(A) and Fig. 9(B) grips the pusher flange 205 of the syringe pusher 202 as illustrated in Fig. 10(B).

When being driven according to an instruction from the control unit 100, the motor 133 of the syringe pusher driving part 7 illustrated in Fig. 4 moves the syringe pusher pushing member 10 in the T-direction by rotation of the feed screw 135. Accordingly, the syringe pusher pushing member 10 can push the syringe pusher 202 in the T-direction and can send the medicinal solution in the syringe body 201 illustrated in Fig. 2 to the patient P through the tube 203 and the indwelling needle 204 accurately.

In the syringe pump 1 of the embodiment of the present invention, when the slider 10 pushes the syringe pusher 202 in the X2-direction (T-direction) and a medicinal agent is sent from the syringe 200, the boot 800 is contracted from the state illustrated in Fig. 11(A) into the state illustrated in Fig. 11(B). When the boot 800 is contracted, the first protruded part 812 and the first protruded part 813 can be stored with the second protruded part 821 placed therebetween being folded. Similarly, the first protruded part 814 and the first protruded part 815 can be stored with the second protruded part 822 placed therebetween being folded. In addition, the remaining second protruded part 823 can be stored, in a folded state, in the first protruded part 816.

As illustrated in Fig. 8, the outer diameter dimension DG1 of the innermost part of the connection part 990 is set smaller than the outer diameter dimension DG2 of the innermost part of the connection part 995. Thus, compared to a case where the outer diameter dimension DG1 of the innermost part of the connection part 990 and the outer diameter dimension DG2 of the innermost part of the connection part 995 are made identical to each other, the first protruded parts 811 and 812 easily become close to each other, the first protruded parts 813 and 814 easily become close to each other, and the first protruded parts 815 and 816 easily become close to each other.

In addition, the outline dimension DG1 of the innermost part of the connection part 990 is set smaller than the outer diameter dimension DG2 of the innermost part of the connection part 995. Thus, even when the boot 800 shrinks as illustrated in Fig. 11 (B) and Fig. 11 (C), the connection part 990 and the connection part 995 do not overlap with each other at the same position and the connection part 995 is placed outside the connection part 990. Accordingly, compared to a case where an overall boot is formed by using first protruded parts, a length of contraction LT can be reduced since a second protruded part can be stored in a folded state in the first protruded part. Since a length of contraction can be reduced when the boot 800 is contracted, a distance to the position where the slider 10 becomes the closest to the side of the housing part 8 can be reduced.

In the embodiment of the present invention, as illustrated in Fig. 11(A) to Fig. 11(C), the second protruded part can be stored in a folded state between the two first protruded parts. Thus, as illustrated in Fig. 11(B), the length of contraction LT in the state in which the boot 800 is the most contracted can be reduced compared to a conventional case where a boot is formed from first protruded parts having the same size. Since it is possible to reduce the length of contraction LT in the state in which the boot 800 is the most contracted, a distance to the position where the slider 10 becomes the closest to the side of the housing part 8 can be reduced.

In addition, when the syringe pusher pushing member 10 which is the moving member of the syringe pusher driving part 7 becomes the closest to the side of the housing part 8, the connection part 990 and the connection part 995 do not overlap with each other since an outer diameter dimension of the connection part 990 between the two successively-formed first protruded parts is smaller than an outer diameter dimension of the connection part 995 between the second protruded part and the first protruded part and a position of the connection part 990 between the first protruded parts and that of the connection part 995 between the second protruded part and the first protruded part are not identical to each other.

Thus, it is possible to reduce the length of contraction LT in the state in which the boot 800 which is the cover member is contracted and to reduce a distance between the syringe pusher pushing member 10 and the housing part 8 of when the syringe pusher pushing member 10 is the closest to the side of the housing part 8. Accordingly, it is possible to reduce a length of the boot 800 in a case where the boot 800 is the most contracted and to reduce a distance between the syringe pusher pushing member 10 of the syringe pusher driving part 7 and the housing part 8 of when the syringe pusher pushing member 10 is the closest to the side of the housing part 8. When the distance between the syringe pusher pushing member 10 and the housing part 8 can be reduced, it is possible to secure a movable amount necessary for the syringe pusher pushing member 10, that is, a movable amount necessary for the syringe pusher, to downsize the extension part 2N of the chassis 2 of the syringe pump 1, and to downsize the chassis 2 of the syringe pump 1.

When a conventional boot in which the same ridge parts are successively formed is used instead of the boot 800 of the embodiment of the present invention, it is not possible to reduce a length of when the boot is the most contracted. Thus, it is not possible to secure a movable amount necessary for the syringe pusher unless a boot in which a longer length in a longitudinal direction is secured is prepared. Accordingly, it becomes necessary to extend the length of the extension part 2N of the chassis 2 of the syringe pump 1. Thus, it is not possible to downsize the chassis.

Note that as a comparison example, when a boot is configured by arranging a large first protruded part and a small second protruded part alternately, the number of small second protruded parts is increased. Thus, it is not possible to adequately secure a length of extension (extension amount) in the X1-direction of the boot. Thus, in the boot of the embodiment of the present invention, two large first protruded parts and one small second protruded part are arranged alternately.

### Second embodiment

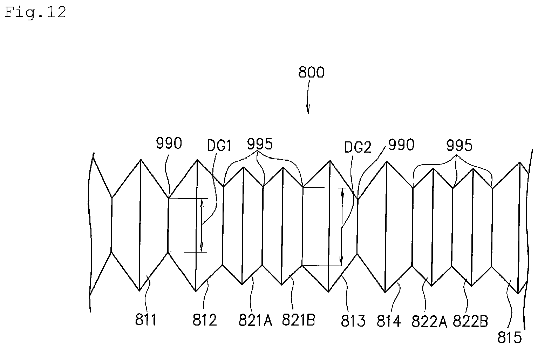

Fig. 12 is a second embodiment of the present invention.

Unlike the case of the boot 800 illustrated in Fig. 8, in a boot 800A of the second embodiment of the present invention which is illustrated in Fig. 12, two second protruded parts 821A and 821B are provided between first protruded parts 812 and 813 and two second protruded parts 822A and 822B are provided between first protruded parts 814 and 815.

A syringe pump 1 of the embodiment of the present invention includes a syringe setting part to set a syringe body of a syringe and a syringe pusher driving part to push a syringe pusher of the syringe. The syringe pusher driving part includes a moving member to grip the syringe pusher detachably and to push out a medicinal agent in the syringe body by moving the syringe pusher relative to the syringe body, a guiding member to move and guide the moving member to a side of the syringe body, and a cover member which covers the guiding member and which can be elastically deformed and contracted as the moving member moves to the side of the syringe body. The cover member includes a plurality of first protruded parts, and at least one second protruded part which is formed between two successively formed first protruded parts and next two successively-formed first protruded parts. An outer diameter dimension of a connection part between the two successively-formed first protruded parts is set smaller than an outer diameter dimension of a connection part between the second protruded part and the first protruded part.

Accordingly, when the cover member is pushed by the moving member and is contracted, the first protruded parts placed on both sides of the second protruded part can be contracted with the second protruded part being folded therebetween. In addition, since an outer diameter dimension of the connection part between the two successively-formed first protruded parts is smaller than an outer diameter dimension of the connection part between the second protruded part and the first protruded part, when the moving member of the syringe pusher driving part becomes the closest to the side of the housing part, the connection part between the first protruded parts and the connection part between the second protruded part and the first protruded part do not overlap with each other. Thus, it is possible to reduce a length of contraction of the cover member in a state in which the cover member is contracted and to reduce a distance between the moving member and the housing part of when the moving member is the closest to the side of the housing part. When the distance between the moving member and the housing part can be reduced, a movable amount necessary for the moving member, that is a movable amount necessary for the syringe pusher can be secured, a chassis of the syringe pump can be downsized, and the syringe pump can be downsized. Thus, by making it possible to reduce a distance between the moving member of the syringe pusher driving part and the housing part of when the moving member is the closest to the side of the housing part by reducing a length of the cover member in a case where the cover member is contracted, it becomes possible to downsize the syringe pump.

One end part of the boot 800 which is included as a cover member is connected to a side surface part of the syringe setting part 8. The other end part of the boot 800 is connected to a side surface of the syringe setting part. Accordingly, it is possible to cover a whole guiding member in such a manner that the side surface part of the syringe setting part and the moving member are connected to each other.

On a side surface of the moving member, a storage part to store at least a part of the contracted cover member is provided. Accordingly, since at least a part of the contracted cover member can be stored in the storage part of the moving member, a distance to a position where the moving member becomes the closest to the side of the housing part can be reduced.

The cover member includes a drip-proof structure. The guiding member is a feed screw which is rotated by an operation of a motor to feed the moving member. Accordingly, the cover member prevents a solution from the outside from attaching to a feed screw.

At an upper part of a body of the syringe pump 1, a display unit 3 to display information and an operation panel unit 4 including an operation button are arranged. At a lower part of a body of the syringe pump 1, a syringe setting part 6 and a syringe pusher driving part 7 are arranged. Accordingly, it is possible for a healthcare professional to perform an operation of sending a medicinal solution from the syringe while checking information on the display unit at the upper part of the body. Then, it is possible for a healthcare professional to operate an operation button on the operation panel unit while checking information on the display unit at the upper part of the body.

The present invention is not limited to the above embodiments and various modifications may be made within the scope of the claims.

### Reference Signs List

1 syringe pump
2 chassis
6 syringe setting part
7 syringe pusher driving part
8V side surface part
10 syringe pusher pushing member (moving member slider)
135 feed screw (example of guiding member)
200, 300, 400 syringe
201, 301, 401 syringe body
202, 302, 402 syringe pusher
205, 305, 405 pusher flange
209, 309, 409 body flange
500 body flange pressing part
800 boot (cover member)
811, 812, 813, 814, 815, 816 first protruded part
821, 822, 823 second protruded part
DG1 outline dimension of connection part 990 between two successively-formed first protruded parts
DG2 outline dimension of connection part 995 between second protruded part and first protruded part

## Claims

1. A syringe pump (1) to which a syringe (200, 300, 400) filled with a medicinal agent is attached and which is configured to send the medicinal agent in the syringe (200, 300, 400) to a patient, comprising:
a syringe setting part (6) configured to set a syringe body (201, 301, 401) of the syringe (200, 300, 400); and
a syringe pusher driving part (7) configured to push a syringe pusher (202, 302, 402) of the syringe (200, 300, 400),
wherein the syringe pusher driving part (7) includes a moving member (10) configured to grip the syringe pusher (202, 302, 402) detachably and to push out the medicinal agent in the syringe body (201, 301, 401) by moving the syringe pusher (202, 302, 402) relative to the syringe body (201, 301, 401), a guiding member (135) configured to move and guide the moving member (10) to a side of the syringe body (201, 301, 401), and a cover member (800) which covers the guiding member (135) and which can be elastically deformed and contracted as the moving member (10) moves to the side of the syringe body (201, 301, 401),
**characterized in that**
the cover member (800) includes protruded parts (811, 812, 813, 814, 815, 816, 821, 822, 823) which are successive in a bellows-shape,
the protruded parts (811, 812, 813, 814, 815, 816, 821, 822, 823) include two first protruded parts (811, 812, 813, 814, 815, 816) which are provided successively in an identical outer diameter dimension and at least one second protruded part (821, 822, 823) which is provided between the first protruded parts (811, 812, 813, 814, 815, 816) and which has an outer diameter dimension smaller than the outer diameter dimension of each of the first protruded parts (811, 812, 813, 814, 815, 816), and
an outer diameter dimension (DG1) of a connection part (990) between the two successively-formed first protruded parts (811, 812, 813, 814, 815, 816) is set smaller than an outer diameter dimension (DG2) of a connection part (995) between the second protruded part (821, 822, 823) and the first protruded part (811, 812, 813, 814, 815, 816).

2. The syringe pump (1) according to claim 1, wherein one end part of the cover member (800) is connected to a side surface part of the syringe setting part (6) and the other end part of the cover member (800) is connected to a side surface of the moving member (10).

3. The syringe pump (1) according to claim 1 or claim 2, wherein on a side surface of the moving member (10), a storage part (88) configured to store at least a part of the contracted cover member (800) is provided.

4. The syringe pump (1) according to any of claim 1 to claim 3, wherein the cover member (800) includes a drip-proof structure and the guiding member (135) is a feed screw (135) to feed the moving member (10) by being rotated by an operation of a motor (133).

5. The syringe pump (1) according to any of claim 1 to claim 4, wherein at an upper part of a body of the syringe pump (1), a display unit (3) configured to display information and an operation panel unit (4) including an operation button are arranged, and at a lower part of the body of the syringe pump (1), the syringe setting part (6) and the syringe pusher driving part (7) are arranged.

## Patentansprüche

1. Spritzenpumpe (1), an welcher eine mit einem Arzneistoff gefüllte Spritze (200, 300, 400) befestigt ist, und welche dazu ausgebildet ist, den Arzneistoff in der Spritze (200, 300, 400) zu einem Patient zu befördern, welche umfasst:
ein Spritzenfassungsteil (6), welches dazu ausgebildet ist, einen Spritzenkörper (201, 301, 401) der Spritze (200, 300, 400) aufzunehmen; und
ein Spritzenkolbenantriebsteil (7), welches dazu ausgebildet ist, einen Spritzenkolben (202, 302, 402) der Spritze (200, 300, 400) zu drücken;
wobei das Spritzenkolbenantriebsteil (7) ein bewegliches Element (10) aufweist, welches dazu ausgebildet ist, den Spritzenkolben (202, 302, 402) abtrennbar zu greifen und den Arzneistoff in dem Spritzenkörper (201, 301, 401) durch Bewegen des Spritzenkolbens (202, 302, 402) relativ zum Spritzenkörper (201, 301, 401) rauszudrücken, ein Führungselement (135), welches dazu ausgebildet ist, das bewegliche Element (10) zu einer Seite des Spritzenkörpers (201, 301, 401) zu bewegen und führen, und ein Abdeckelement (800), welches das Führungselement (135) abdeckt und welches elastisch deformiert und kontrahiert werden kann, während das bewegliche Element (10) sich zu der Seite des Spritzenkörpers (201, 301, 401) bewegt,
**dadurch gekennzeichnet, dass**
das Abdeckelement (800) vorstehende Teile (811, 812, 813, 814, 815, 816, 821, 822, 823) aufweist, welche aufeinanderfolgend in Balgform angeordnet sind,
die vorstehenden Teile (811, 812, 813, 814, 815, 816, 821, 822, 823) zwei erste vorstehende Teile (811, 812, 813, 814, 815, 816) aufweisen, welche aufeinanderfolgend mit identischen Außendurchmessern bereitgestellt sind, und mindestens ein zweites vorstehendes Teil (821, 822, 823), welches zwischen den ersten vorstehenden Teilen (811, 812, 813, 814, 815, 816) bereitgestellt ist und welches einen kleineren Außendurchmesser als die ersten vorstehenden Teile (811, 812, 813, 814, 815, 816) hat, und
ein Außendurchmesser (DG1) eines Verbindungsteils (990) zwischen den zwei aufeinanderfolgend-gebildeten ersten vorstehenden Teilen (811, 812, 813, 814, 815, 816) kleiner als ein Außendurchmesser (DG2) eines Verbindungsteils (995) zwischen dem zweiten vorstehenden Teil (821, 822, 823) und dem ersten vorstehenden Teil (811, 812, 813, 814, 815, 816) ist.

2. Spritzenpumpe (1) nach Anspruch 1, wobei ein Endteil des Abdeckelements (800) mit einem Seitenflächenteil des Spritzenfassungsteils (6) verbunden ist und das andere Endteil des Abdeckelements (800) mit einer Seitenfläche des beweglichen Elements (10) verbunden ist.

3. Spritzenpumpe (1) nach Anspruch 1 oder 2, wobei auf einer Seitenfläche des beweglichen Elements (10) ein Aufbewahrungsteil (88) bereitgestellt ist, welches dazu ausgelegt ist, mindestens einen Teil des kontrahierten Abdeckelements (800) aufzubewahren.

4. Spritzenpumpe (1) nach einem der Ansprüche 1 bis 3, wobei das Abdeckelement (800) einen tropfsicheren Bereich aufweist und das Führungselement (135) eine Zuführschnecke (135) ist, um das bewegliche Element (10) durch Rotation mittels eines Motors (133) voranzutreiben.

5. Spritzenpumpe (1) nach einem der Ansprüche 1 bis 4, wobei an einem oberen Teil eines Körpers der Spritzenpumpe (1) eine zur Anzeige von Informationen ausgelegte Anzeigeeinheit (3) und eine einen Bedienknopf aufweisende Bedienfeldeinheit (4) angeordnet sind, und wobei an einem unteren Teil des Körpers der Spritzenpumpe (1) das Spritzenfassungsteil (6) und das Spritzenkolbenantriebsteil (7) angeordnet sind.

## Revendications

1. Pompe à seringue (1) à laquelle une seringue (200, 300, 400) remplie d'un agent médicinal est fixée et qui est conçue pour injecter l'agent médicinal contenu dans la seringue (200, 300, 400) à un patient, comprenant :
une partie de fixation de seringue (6) conçue pour fixer un corps de seringue (201, 301, 401) de la seringue (200, 300, 400) ; et
une partie de commande de piston de seringue (7) conçue pour pousser un piston de seringue (202, 302, 402) de la seringue (200, 300, 400),
dans laquelle la partie de commande de piston de seringue (7) comprend un élément mobile (10) conçu pour saisir le piston de seringue (202, 302, 402) de façon détachable et pour expulser l'agent médicinal contenu dans le corps de seringue (201, 301, 401) en déplaçant le piston de seringue (202, 302, 402) par rapport au corps de seringue (201, 301, 401), un élément de guidage (135) conçu pour déplacer et guider l'élément mobile (10) vers un côté du corps de seringue (201, 301, 401), et un élément de protection (800) qui couvre l'élément de guidage (135) et qui peut être élastiquement déformé et contracté lorsque l'élément mobile (10) se déplace vers le côté du corps de seringue (201, 301, 401),
**caractérisée en ce que**
l'élément de protection (800) comporte des parties faisant saillie (811, 812, 813, 814, 815, 816, 821, 822, 823) qui se succèdent sous une forme de soufflet,
les parties faisant saillie (811, 812, 813, 814, 815, 816, 821, 822, 823) comportent deux premières parties faisant saillie (811, 812, 813, 814, 815, 816) qui sont disposées de manière successive dans une dimension de diamètre extérieur identique et au moins une seconde partie faisant saillie (821, 822, 823) qui est placée entre les premières parties faisant saillie (811, 812, 813, 814, 815, 816) et qui a une dimension de diamètre extérieur inférieure à la dimension de diamètre extérieur de chacune des premières parties faisant saillie (811, 812, 813, 814, 815, 816), et
une dimension de diamètre extérieur (DG1) d'une partie de connexion (990) entre les deux premières parties faisant saillie (811, 812, 813, 814, 815, 816) formées de manière successive est réglée pour être inférieure à une dimension de diamètre extérieur (DG2) d'une partie de connexion (995) entre la seconde partie faisant saillie (821, 822, 823) et la première partie faisant saillie (811, 812, 813, 814, 815, 816).

2. Pompe à seringue (1) selon la revendication 1, dans laquelle une partie d'extrémité de l'élément de protection (800) est reliée à une partie de surface latérale de la partie de fixation de seringue (6) et l'autre partie de l'élément de protection (800) est reliée à une surface latérale de l'élément mobile (10).

3. Pompe à seringue (1) selon la revendication 1 ou la revendication 2, dans laquelle sur une surface latérale de l'élément mobile (10), est prévue une partie de stockage (88) conçue pour loger au moins une partie de l'élément de protection (800) contracté.

4. Pompe à seringue (1) selon l'une quelconque des revendications 1 à 3, dans laquelle l'élément de protection (800) comprend une structure étanche aux gouttes et l'élément de guidage (135) est une vis de commande de l'avance (135) destinée à faire avancer l'élément mobile (10) en étant mise en rotation par l'actionnement d'un moteur (133).

5. Pompe à seringue (1) selon l'une quelconque des revendications 1 à 4, dans laquelle au niveau d'une partie supérieure d'un corps de la pompe à seringue (1), une unité d'affichage (3) conçue pour afficher des informations, et une unité de panneau de commande (4) comportant un bouton de commande sont disposées, et au niveau d'une partie inférieure du corps de la pompe à seringue (1), la partie de fixation de seringue (6) et la partie de commande de piston de seringue (7) sont prévues.
